(19)

Europäisches
Patentamt

European
Patent Office

Office européen
des brevets

(11)     **EP 3 532 892 B1**

(12)                    **EUROPEAN PATENT SPECIFICATION**

(45) Date of publication and mention
of the grant of the patent:
**23.04.2025  Bulletin 2025/17**

(21) Application number: **16825546.1**

(22) Date of filing: **28.10.2016**

(51) International Patent Classification (IPC):
*A61B 3/08* (2006.01)          *G02C 7/08* (2006.01)
*A61B 3/028* (2006.01)         *A61B 3/02* (2006.01)
*A61B 3/00* (2006.01)          *G02B 27/01* (2006.01)

(52) Cooperative Patent Classification (CPC):
**A61B 3/028; A61B 3/08; G02B 27/017;
G02B 27/0172; G02C 7/08;** G02B 2027/0127;
G02B 2027/0132; G02B 2027/0178;
G02B 2027/0185

(86) International application number:
**PCT/IB2016/001705**

(87) International publication number:
**WO 2018/078409 (03.05.2018 Gazette 2018/18)**

(54) **METHOD OF DETERMINING AN EYE PARAMETER OF A USER OF A DISPLAY DEVICE**

VERFAHREN ZUR BESTIMMUNG EINES AUGENPARAMETERS EINES BENUTZERS EINER
ANZEIGEVORRICHTUNG

METHOD OF DETERMINING AN EYE PARAMETER OF A USER OF A DISPLAY DEVICE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**04.09.2019  Bulletin 2019/36**

(73) Proprietor: **Essilor International
94220 Charenton-le-Pont (FR)**

(72) Inventors:
• **MARIN, Gildas
94227 Charenton-le-Pont (FR)**
• **HERNANDEZ-CASTANEDA, Martha
94227 Charenton-le-Pont (FR)**

• **BARANTON, Konogan
94227 Charenton-le-Pont (FR)**
• **FERMIGIER, Bruno
94227 Charenton-le-Pont (FR)**
• **BOUCHIER, Aude
94227 Charenton-le-Pont (FR)**
• **SAHLER, Jean
94227 Charenton-le-Pont (FR)**

(74) Representative: **Cabinet Novitech
188 Grande rue Charles de Gaulle
94130 Nogent-sur-Marne (FR)**

(56) References cited:
WO-A1-2016/149416      US-A1- 2004 212 776
US-A1- 2013 127 821      US-A1- 2013 285 885
US-A1- 2014 362 110

EP 3 532 892 B1

**Description**

FIELD OF THE INVENTION

The invention relates to a method for determining an eye parameter of a user of a display device, the eye parameter relating to a dioptric parameter of an ophthalmic lens to be provided to the user.

BACKGROUND OF THE INVENTION

Usually, a person wishing to have an optical equipment goes to see an eye care practitioner.

The eye care practitioner orders the eyewear equipment at an optical lab by sending an order request to the optical lab. The order request may comprise wearer data, for example the wearer's prescription, fitting data, spectacle frame data, for example the type of spectacle frame the wearer has selected, and lens data, for example the type of optical lens the wearer has selected.

The determination of the wearer's prescription and fitting data may require carrying out complex and time consuming measurements. Such measurements usually require complex and costing material and qualified personnel to be carried out.

The usual methods for determining the dioptric parameters of an ophthalmic lens to be provided to a person have a number of drawbacks among which the difficulty to control accurately the person's eye accommodation. Furthermore, the usual methods are usually distressing for the person and therefore are usually not carried out over a long period of time. Thus the results provided by such methods fail to provide indication of long term visual comfort of the person.

In addition, the usual methods are carried out in a controlled environment, for example the eye care practitioner's office and not in real life conditions.

Finally, implementing such methods for determining binocular vision increases the difficulty and length of the method making such implementation even more distressing for the person.

A document of the prior art disclosing a method and a system for determining the dioptric parameters of an ophthalmic lens to be provided to a user is WO2016/149416 A1.

Therefore, there is a need for a method for determining the dioptric parameter of an ophthalmic lens to be provided to the user without going through the classic refractive means such as autorefractor, phoropter, testing glasses, optometrist or ophthalmologist).

One object of the present invention is to provide such method.

SUMMARY OF THE INVENTION

To this end, the invention proposes a method for determining an eye parameter of a user of a display device, the eye parameter relating to a dioptric parameter of an ophthalmic lens to be provided to the user, according to claim 1.

Advantageously, the method of the invention allows the user himself determining the dioptric parameter of an ophthalmic lens to be provided to him using a simple and playful method. The user may further order an ophthalmic lens based on the determined dioptric parameter.

The method of the invention may also be used to adjust the parameters of a binocular display device so as to provide clear vision to the user.

According to further embodiments which can be considered alone or in combination:

- the binocular display device is configured to displaying images towards the two eyes of the user having both independent features and common features; and/or
- the display device comprises moveable lenses through which the user sees the images displayed and during the display parameter modifying step the lenses are moved so as to modify the virtual display distance of the perceived image; and/or
- the display device comprises a light field display and during the display parameter modifying step at least one parameter of the light field display is modified so as to modify the virtual display distance of the perceived image; and/or
- the display device has means for selecting a virtual display distance among a set of predetermined virtual distances, and the virtual display distance of the perceived image is obtained from the use of at least one of the predetermined distance; and/or
- the display device has an optical element for example lens and/or mirror, having adjusting means to adjust the power of the optical element; and/or
- the binocular display device is a head mounted binocular display device; and/or
- the image subjective quality relates to the sharpness of the perceived image; and/or
- the method of the invention further comprises an astigmatism determining step during which an image or a plurality of images comprising similar elements with different orientations are displayed to the user and the orientations corresponding to the subjective sharpest perceived images are determined; and/or

- the perceived image subjective quality relates to the contrast of the perceived image; and/or
- the image display step and the display parameter modifying step are implemented in monocular vision; and/or
- the method of the invention further comprises an eye tracker providing step during which an eye tracker is provided and during the image display step the eye tracker is used to determine if the user is seeking in peripheral or central vision; and/or
- the method of the invention further comprises an accommodation relaxation step during which the user's accommodation is relaxed; and/or
- the method of the invention further comprises a calibration step during which a correlation between the at least one parameter of the binocular display device modified during the display parameter modifying step and the virtual display distance is determined, said correlation is used during the eye parameter determining step to determine the eye parameter of the user.

BRIEF DESCRIPTION OF THE DRAWINGS

Embodiments of the invention will now be described, by way of example only, and with reference to the following drawings in which:

- Figure 1 is a flow chart representing a method for determining an eye parameter of a user of a display device according to the invention,
- Figures 2 and 3 illustrate binocular display devices that may be used in the method of the invention,
- Figure 4 illustrates convergence angle $\alpha$,
- Figures 5A and 5B illustrates an implementation of a display parameter modifying step according to the invention, and
- Figures 6, 7A and 7B shows an example of implementation of a calibration step.

DETAILED DESCRIPTION OF PREFERRED EMBODIMENTS

Elements in the figures are illustrated for simplicity and clarity and have not necessarily been drawn to scale. For example, the dimensions of some of the elements in the figure may be exaggerated relative to other elements to help improve the understanding of the embodiments of the present invention.

The invention relates to a method for determining an eye parameter of a user of a display device, the eye parameter relating to a dioptric parameter of an ophthalmic lens to be provided to the user.

In the sense of the invention, dioptric parameters are parameters defining the dioptric function of an optical lens.

The dioptric function corresponds to the optical lens power (mean power, astigmatism etc... ) as a function of the gaze direction.

The wording "optical design" is a widely used wording known from the man skilled in the art in ophthalmic domain to designate the set of parameters allowing to define a dioptric function of an ophthalmic lens; each ophthalmic lens designer has its own designs, particularly for progressive ophthalmic lenses. As for an example, a progressive ophthalmic lens "design" results of an optimization of a progressive surface so as to restore a presbyope's ability to see clearly at all distances but also to optimally respect all physiological visual functions such as foveal vision, extra-foveal vision, binocular vision and to minimize unwanted astigmatisms. For example, a progressive lens design comprises:

- a power profile along the main gaze directions (meridian line) used by the lens wearer during day life activities,
- distributions of powers (mean power, astigmatism,...) on the sides of the lens, that is to say away from the main gaze direction.

These optical characteristics are part of the "designs" defined and calculated by ophthalmic lens designers and that are provided with the progressive lenses.

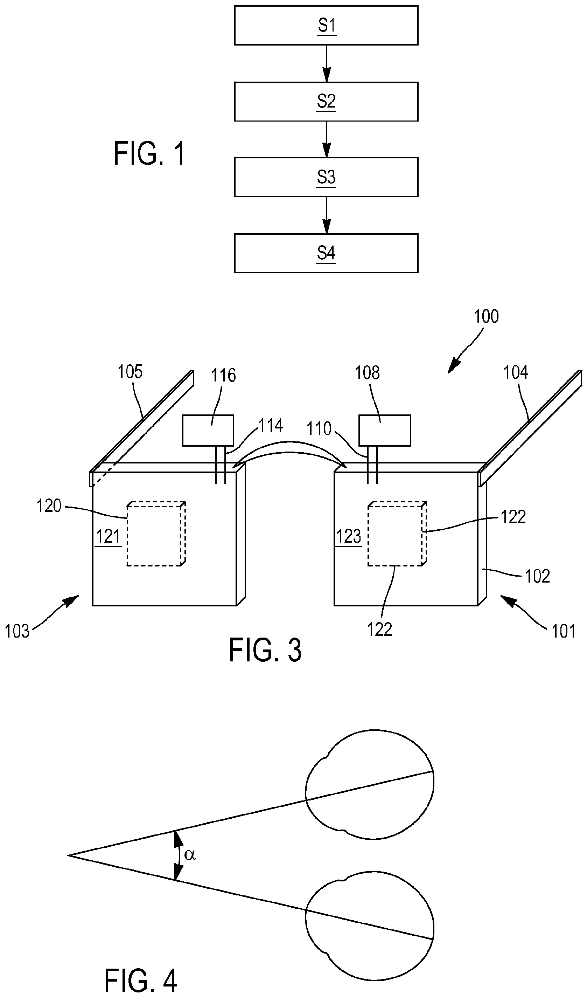

As illustrated on figure 1, the method according to the invention comprises at least:

- a display device providing step S1,
- an image display step S2,
- a display parameter modifying step S3, and
- an eye parameter determining step.

A binocular display device is provided to the user during the display device providing step S1.

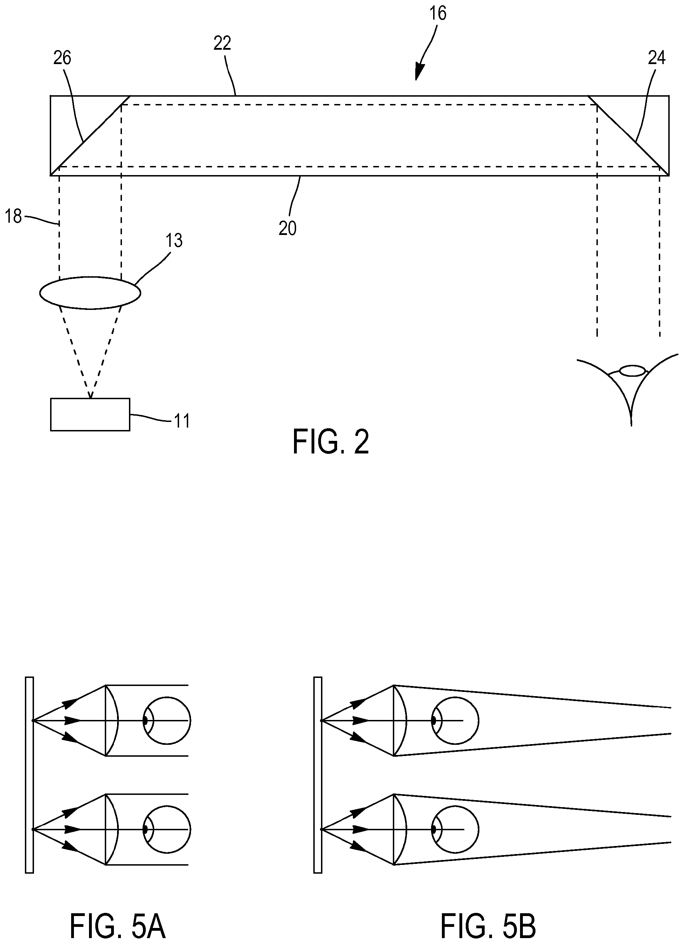

As represented on figure 2, the display device according to the invention may preferably comprise a see-through display system, allowing the wearer to see both the virtual image and the real world through it. The see-through display system is able to display graphical images, and an electronic driving system (memory + processor) sends to the display system the image to display. Preferably it is able to display image in different viewing directions. Furthermore, the image to be displayed can be modified.

An example of see-through display system is illustrated in figure 2. Such see-trough display system comprises a display source 11, a collimating device 13, and an optical insert 16 constituted by a light-guide optical element 16 (LOE).

The display source 11 can be emissive or not emissive.

It can be directly obtained from either a spatial light modulator (SLM) such as a cathode ray tube (CRT), a liquid crystal display (LCD), an organic light emitting diode array (OLED), liquid crystal on silicon (LCoS) or similar devices, or indirectly, by means of a relay lens or an optical fiber bundle. The display source 11 comprises an array of elements (pixels) imaged to infinity by the collimating device 13, for example a collimating lens.

The light-guide optical element 16 typically includes at least two major surfaces 20 and 22 and edges, at least one partially reflecting surface 24 and an optical element 26 for coupling light thereinto. The output waves 18 from the collimating device 13 enter the light-guide optical element 16 through its lower surface 20. The incoming waves (towards the light-guide optical element 16) are reflected from the surface 26 and trapped in the light-guide optical element 16.

In an embodiment, the see-through display system may comprise a plane light-guide optical element 16 with at least two planes major surfaces 20 and 22. For example, such a light guide optical element 16 may be one of Lumus Company.

In an alternative embodiment, the see-through display system may comprise a curved light-guide optical element 16.

The light-guide may be encapsulated in an optical lens or placed in front of an optical lens.

According to the method of the invention, the display device is a binocular head mounted device as represented on figure 3.

In this example, the display device comprises a frame. The frame may be similar to a conventional eyeglasses frame and can be worn with a similar comfort level. However, other implementations are possible, such as a face shield which is mounted to the user's head by a helmet, strap, hold by the user himself, or other means, for example VR HMD such as the Rift, the Gear VR or the DK2 from the company Oculus or the Google cardboard.

The frame includes a frame front 102 and temples 104 and 105. The frame front holds a see-through lens 101 for the user's left eye and a see-through lens 103 for the user's right eye. The left and right orientations are from the user's perspective.

The left-side see-through lens 101 includes an optical component 122 such as a beam splitter which mixes an augmented reality image with light from the real-world scene for viewing by the left eye.

The right-side see-through lens 103 includes an optical component 120 such as a beam splitter which mixes an augmented reality image with light from the real-world scene for viewing by the right eye.

A right-side augmented reality emitter 116 is mounted to the frame via an arm 114, and a left-side augmented reality emitter 108 is mounted to the frame via an arm 110.

An electrical power source, for example a battery, provides power to the different elements of the head mounted device.

Appropriate electrical connections can be made via conductive paths in the frame, for instance.

Component 122 and 120 may also include a dimmer that can be an electro active dimmer, such as an electrochromic device, or a liquid crystal dimmer.

This dimmer may be active so as to block light coming from external environment during the image display step S2, allowing the user not to be disturb and only perceive virtual image. This ensures that contrast is maximum and so that wearer's prescription is determined with accuracy.

During the image display step S2, an image is displayed to the user when using the display device.

The images displayed to the eyes of the user may be the same for both eyes or different.

According to the method of the invention, the binocular display device is configured to display independently images towards the two eyes of the user.

Furthermore, the binocular display device may be configured to display images towards the two eyes of the user having both independent features and common features.

The binocular display device may be configured to display similar features which can be different in some conditions and common in other ones. For example, the display distance can be the same, but the convergence angles of the two images are different, such configuration is advantageous for instance for conducting wearer's refraction at particular distance (for instance near distance).

According to a further example, the distances are different, but convergence angles are the same, such configuration is advantageous when wearer's prescription is different between the left and right eyes.

As illustrated on figure 4, convergence angle $\alpha$ is the horizontal angle between a reference direction (straight forward preferably) and the gaze direction of the eye of the wearer. When looking at far, convergence angles are similar for both eyes, while when looking at near, gaze direction for both eye will intersect at near, and thus are not similar.

At least one display parameter of the binocular display device is modified during the display parameter modifying step S3 so as to modify the virtual display distance of the perceived image.

This can be achieved in different ways: displays generally have an adjustment lens between the eye and the display to compensate for the short distance and return the image to infinity. The correction of the eye can be adjusted either by using a variable focal lens or by adjusting the position of the lens between the eye and the display.

According to an embodiment of the invention, the display device comprises moveable lenses through which the user sees the images displayed and during the display parameter modifying step S3 the lenses are moved so as to modify the virtual display distance of the perceived image.

Such variation is ideally continuous, but can also be carried in a discrete manner, for example in steps of 0.25D.

In the case of a display device with movable lenses, the displacement of the lenses allows to vary the distance D of the virtual image as illustrated on figures 5A and 5B.

When the lenses are positioned such that the focal point is on the display, the virtual image is then seen at infinity as illustrated on figure 5A. Whereas when the lens is moved away from the previous position, the wavefront is convergent (D> 0) which is like putting a positive power before the user's eye, as illustrated on figure 5B.

According to a further embodiment of the invention, the display device may comprise a light field display. During the display parameter modifying step 3 at least one parameter of the light field display is modified so as to modify the virtual display distance of the perceived image.

The display device may comprise means for selecting a virtual display distance among a set of predetermined virtual distances, and the virtual display distance of the perceived image is obtained from the use of at least one of the predetermined distance. An example of such display device is disclosed in US20150016777.

According to an embodiment, the display device has an optical element having adjusting means to adjust the power of the optical element or liquid crystal lenses, for example varioptic or membrane lenses for example optotune. In an alternative, the display means may also display holographic images using a light source and a SLM. The SLM may be programmed so as to change the distance of the virtual image using well known computer-generated holography techniques.

The display parameter modifying step s3 is repeated until the subjective quality of the perceived image is perceived by the user as optimal.

The subjective quality of the perceived image may relate to the sharpness or to the contrast of the perceived image.

The display image and display parameter modifying steps may be implemented in monocular vision. In other words, having the user use only one eye, for example by closing the other eye, or having black image displayed for the other eye.

The display image and display parameter modifying steps may be implemented in binocular vision. In other words, having the user use both eyes and displaying images to both eyes of the user. This is a preferred solution to prevent the user to use eye accommodation that would lead to inaccurate refraction.

The method of the invention uses the possibilities of separate display (different images) between the two eyes of a binocular display device, such as a virtual reality headset, to achieve a refractive binocular vision, possibly at home, and allow testing the quality and problems of binocular vision.

The method of the invention enables carrying out tests in a more friendly or ecological situation.

The method of the invention allows testing a dioptric parameter at home and/or over a long period of time.

For determining spherical power, the image displayed during the image display step may correspond to optotypes or Duochrome tests. In case of use of optotypes, the image displayed giving highest acuity will be determined, while in case of use of duochrome test, the image displayed giving balanced contrast or sharpness between red/green target will be determined.

The method of the invention may further comprise an astigmatism determining step.

During the astigmatism determining step an image or a plurality of images comprising similar elements with different orientations are displayed to the user and the orientations corresponding to the subjective sharpest perceived images are determined. This will provide the orientation of the axis of cylinder.

The cylindrical parameters, power and orientation, may optionally be determined by using the rotating cylinder method, or more simply, it can be determined using a Duochrome test oriented along the axis of correction to successively determine the two focal lengths for each eye of the user. The cylinder may further be determined by using a test sharpness / acuity measurement in a preferred orientation.

This is particularly advantageous in the sense that most display devices offer no way to generate a wave front with astigmatism, but are limited to change the distance between the virtual object and the wearer of the device.

To determine the refraction in the presence of cylinder one can also determine the cylinder axis using a Parent test and then displaying to the user images oriented along this axis and orthogonally (for instance using Gabor patterns), the spatial frequency of the displayed images corresponds for example to an acuity of 10/10.

The images may consist of periodic frames along the axis, the virtual display distance being chosen so that the image is seen blurred by the user even with accommodation, for example the distance Dinitiale is chosen such that 1 / DinitialAxe = PuserAxe + 1D PuserAxe with the user's prescription along the axis in question.

The distance is then changed so as to be closer to the Punctum Remotum. When the user perceives the image, or he considers having a clear vision of the image, it means that 1 / Daxe = PuserAxe. The same is done for a stimulus in an orthogonal axis: 1 / DAxeOrtho = PuserOrtho
We deduce the refraction:

$$S = PuserAxe, C = PuserOrtho-PuserAxe , and AXE = axis \quad (1)$$
Or
$$S = PuserOrtho , C = PuserAxe - PuserOrtho and AXE = axis + 90°$$

When performing tests involving binocular vision and measuring the dioptric parameter for distance vision, it is preferable that the light beams entering the right eye and the left and corresponding to the same points left and right eye picture are parallel to avoid seeking the convergence of the eyes of the user, in other word to keep convergence angle parallel between left and right eye. In other words, it is preferable that the distance between the right and left virtual images correspond to the interpupillary distance of the wearer when conducting wearer's refraction at far.

Thus, according to the method of the invention, the method further comprises a distance adjustment step during which the distance between a right reference point of the right image to be seen by the right eye and a left reference point of the left image to be seen by the left eye is adjusted so that right reference point and left reference point provide parallel beams to the user.

Furthermore, during the distance adjustment step the distance between the right reference point and the left reference point is adjusted based on the virtual convergence distance so as to keep convergence angle parallel for left and right eye, whatever is the position of the movable lenses. This must be done particularly when the distance between eye pupils differs from the distance between left and right lens optical centers.

In practice, in the case of movable lenses or variable power lenses, this may be achieved by determining optical path by ray tracing: determining a ray exiting from the right eye, horizontal, and is propagated through the lens and its impact on the right display, Point R, is determined. The same is performed for the left eye, Point L.

The left and right images must then be separated on the right and left displays by a distance corresponding to the distance Point R - Point G which allows not having convergence issues ie convergence angle are parallel for both eyes and whatever the interpupillary distance of the user may be.

If this convergence correction is not made, a convergence appears that may distort the result of the method, because of the link between accommodation and convergence that will create accommodation to the user. In the particular case where the optical center distance between the left and right lenses equals the pupil distance, the distance between the right and left images must also correspond to the distance between the optical axes of right and left lenses.

These remarks are valid regardless of the display device for example mobile lenses, using Light Field display for displaying images at different distances, power lenses, holographic image, although the correction may be different.

For a holographic image, or an image displayed by light field display, the right and left images may be displayed so that the distance between them is directly the pupillary distance.

In practice the pupillary distance of the user can be determined via an eye tracker positioned in the display device, or using a secondary measurement means, such as a smartphone, a tablet to take the picture of the user's eyes and measure the distance between the pupils.

Moving the left and right lenses with respect to the displays generates a variation in angular size that is best to be corrected.

Therefore, according to the method of the invention, the method further comprises a scaling step during which the right and left images are scaled so that both images are seen with the same angular size by the user.

The angular size of an object displayed is, in approximation, $\Phi$ = atan (Size / Distance) with Size the size of the object on the display and Distance the distance between the display and the right or left lens and the angular size $\Phi$.

It is therefore preferable to correct that size so that the right and left images are views of the same angular size and that the angular sizes displayed correspond to the desired angular sizes for example 1min of arc. The angular size being known, the distance being also known, it is then easy to derive the size to be displayed. The distance may be known for instance from position coder inside the device when using movable lens, or from a calibration step (see below).

The method of the invention further comprises a depth perception test step during which different images at different distances from a reference position are provided, and for each image it is checked if the user can detect if the distance is shorter or greater that the reference position.

The binocular vision of the display device simulates changes of proximity of the convergence plan that can permit the measurement of wearer's refraction at near distance. To do so, object to be seen are displayed at different positions in the different image to each of the two eyes. These positions are determined so that convergence angle is the convergence angle that right/left eye would adopt when looking at near. For instance, to simulate an object being at 330mm, convergence angle may be -atan(0.5Pd/330) for right eye and +atan(0.5Pd/330) for left eye. The virtual distance is also changed for each eye using step 3, so that the user can see clearly at near.

The dioptric parameters may be different in central and peripheral vision. Usually the myopia and astigmatism are greater in peripheral vision.

Therefore, when carrying out the method of the invention it may be preferable to determine if the user is in central or peripheral vision. This is to avoid under / over correction which will cause visual fatigue or discomfort. When the central or peripheral vision is controlled determining the dioptric parameters is more accurate.

The method of the invention may therefore, comprise an eye tracker providing step during which an eye tracker is provided and during the image display step the eye tracker is used to determine if the user is looking in peripheral or central vision.

Upon carrying out the method of the invention, an issue may be to control the accommodation variations of the user. This is to avoid under / over correction which will cause visual fatigue or discomfort. When the accommodation is well controlled measuring the dioptric parameters is more accurate.

Therefore, the method of the invention may further include an accommodation relaxation step during which the user's accommodation is relaxed.

The accommodation may be relaxed using specific images displayed to the user, for example by displaying images that provide the visual impression of diverging or image in perspective. The accommodation may be relaxed by having the user listen to music or relaxing sound or looking at a releasing video.

To provide accurate measurements, the display device used in the method of the invention may need to be calibrated.

Therefore, the method further comprises a calibration step during which a correlation between the at least one parameter of the binocular display device modified during the display parameter modifying step and the virtual display distance is determined. The correlation is used during the eye parameter determining step to determine the eye parameter of the user.

For example, the display distance may need to be determined with a greater accuracy than normally allowed by usual binocular display devices.

Most binocular display devices do not allow determining the display distance accurately because they are not designed to measure refractive error at 0.25D. Existing standards on dioptric parameter such as ISO10341 on refraction provide for 0.06D tolerances for a refraction between 0 and 3D.

The method therefore comprises a calibration step to allow determining accurately such distance. The calibration step may be implemented after the eye parameter determining step or prior to the display device providing step.

The calibration step may comprise the user of a device like the smartphone / tablet incorporating a camera.

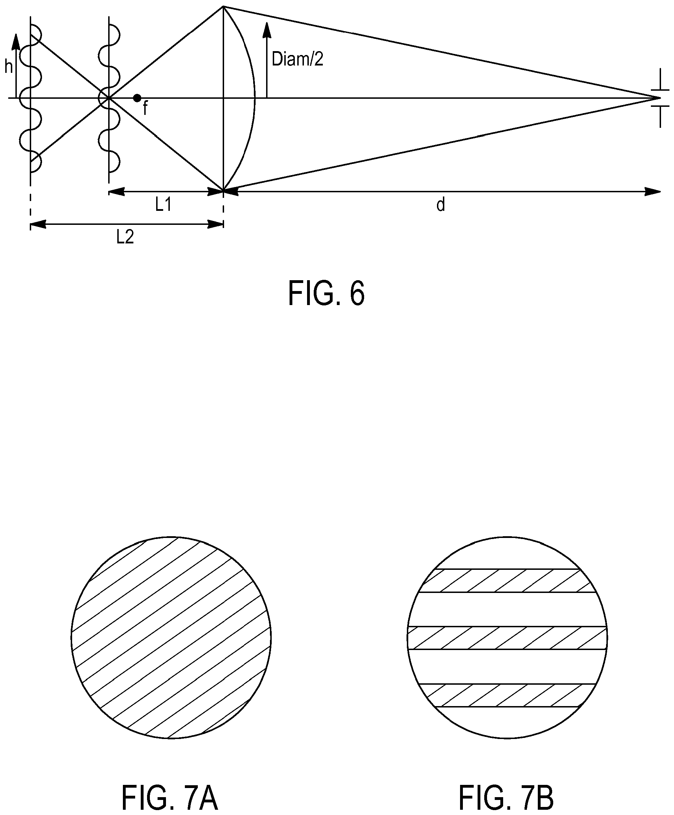

Figure 6 shows an example of implementation of a calibration step, where the display is movable relative to a lens for imaging the display at a varying distance.

A sinusoidal horizontal frame type is displayed. A smartphone is used to acquire images of the display with the smartphone camera

On figure 6, two possible display positions are represented, a first position at a distance L1 of the lens, and a second position at a distance L2 of the lens.

At the first distance L1, the display and the lens of the smartphone's camera are conjugates: a flat uniform color is acquired by the camera, this color extends over the surface of the lens as illustrated on figure 7A.

At the second distance L2, there is no conjugation, and one can observe a numbers of fringes as illustrated on figure 7B. This second position corresponds to the position for which the user sees a sharp image, and thus compensates for his prescription.

One may, based on the number of fringes, determine the viewing distance of the virtual image and therefore the spherical power.

Indeed, with reference to figure 6, one may consider:
$CL1 + Cf = Cd$, with $CL1 = 1 / L1$ ($L1$ is $<0$), $Cf = 1 / f$, with $f$ the lens focal distance and $Cd = 1 / d$ with $d$ the distance between the smartphone and the lens (positive)

We also have $(L2-L1) / h = L1 / (Diam / 2)$, with $Diam$ with the diameter of the lens and $h$ the height on the display corresponding to the visible sinusoid

Therefore, $CL2 = CL1 / (1 + h / (Diam / 2))$ and $Pref = CL2 + Cf$, with $Pref = CL1 / (1 + h / (Diam/2)) + Cf$
Based on the first relationships we get gives $CL1 = Cd-Cf$ and $Pref = (Cd-Cf)/ (1 + h / (Diam/ 2)) + Cf$
It is assumed $h$ small before $Diam$, which gives at the first order:

$$Pref = (cd-Cf) * (1-h / (Diam / 2) + Cf = Cd + Cf * h / (Diam / 2)$$

Cd may be estimated or measured, for example, if one takes a picture at a distance of 1m, Cd = 1D. If one moves away from 4m, Cd = 0.25D. Beyond the influence of Cd becomes negligible.

Cf is a data of the lens, for example a focal length of 5cm Cf = 20D.

The height h is determined by processing the picture and determining the number of visible fringe to the edge, and knowing the number of pixels used to display these fringes and size of pixels, one may have: h = nbfranges *nbpixel_per_franges * size_pixel.

Therefore, it is possible, by measuring through the smartphone the number of fringe, determining the distance of the virtual image and thereby the user's ametropia. Preferably, the distance between the smartphone and the display device is important (>=2m) so as to improve accuracy.

The spherical power is determined from the number of fringes, and the proximity of the smartphone is added to the result. For instance, when no fringes are visible (fig.7A), power is 0D, and final result is 0D+0.5D when the distance between smartphone and display device is 2m (0.5D=1/2m).

When trying to measure astigmatism, fringes appear following two directions along the lines of the cylinder axis, and one may determine two distances and associated powers.

If the display device used allows to obtain an indication of the correction setting, for example an index on the lens position or a number corresponding to the corrective lens used, but does not have the distance accurately, the inaccuracy may be relatively constant as long as we are using a model of the same brand.

In this case, one just needs to determine the average default beforehand for each model and for each configuration or index value proposed. With the knowledge of the model of display device used, one may convert the indication of correction used in a virtual distance to accurately adjust based on the average defect observed for this type of model.

To determine the correction to be applied, one may make the above mentioned measurements for each model of display devices.

Furthermore, one can use more accurate means, for example replace the camera of the smartphone with a high resolution camera aligned on an optical bench or measure the power values corrective lenses using frontofocometers or HartmannShack analyzer or any other means of known measure.

One can measure the positioning of the corrective lenses relative to the display accurately, for example by means of a measuring device or micrometric mechanical gauge or a laser range finder or other vector system suitable for accurately measuring a distance.

The idea here is not to be limited by the means of measurement as these measures are carried out beforehand in the laboratory or in the factory and not by the end user.

A correlation table for most, for example all, of the binocular display device models available may be established.

The correction table may be directly accessible over the internet, the user himself providing the value of the index or lens number used.

The conversion can be accessed as a web app or smartphone that converts the initial value in an ophthalmic correction value. The application could even directly order the necessary corrective lenses.

If the binocular display device permits, the index may be directly read and interpreted by a suitable application that has access to the conversion table or directly embedded in the application, or by querying an Internet server by providing this conversion table.

The inventors propose a second solution to measure viewing distance of the virtual image with a camera and a measuring tape.

The method consists in adjusting the focus of the camera on the virtual image and taking a picture of a calibrated pattern indicating the distance of focus.

First, a camera is placed in front of the binocular display device as if it was the eye of the user.

Ideally, the pupil of the camera is in the same position as would the pupil of the user be. The focus of the camera is adjusted on the virtual image in autofocus mode, and hold.

Secondly, the binocular display device is removed and a measuring tape is placed, with the zero mark at the same position as would be the user's eye. The camera then takes a picture with the distance of focus unchanged.

In the photo, it is easy to read the focus distance. An analysis of the image by image processing and adjustment of the camera with a shallow depth of field allows increasing the accuracy of the measurement.

The invention has been described above with the aid of embodiments without limitation of the general inventive concept; in particular the mounted sensing device is not limited to a head mounted device.

Many further modifications and variations will suggest themselves to those skilled in the art upon making reference to the foregoing illustrative embodiments, which are given by way of example only and which are not intended to limit the scope of the invention, that being determined solely by the appended claims.

In the claims, the word "comprising" does not exclude other elements or steps, and the indefinite article "a" or "an" does not exclude a plurality. The mere fact that different features are recited in mutually different dependent claims does not indicate that a combination of these features cannot be advantageously used. Any reference signs in the claims should not be construed as limiting the scope of the invention.

**Claims**

1. Method for determining an eye parameter of a user of a display device, the eye parameter relating to a dioptric parameter of an ophthalmic lens to be provided to the user, the method comprising:

   - a display device providing step, during which a binocular display device is provided to the user,
   - an image display step, during which an image is displayed to the user when using the display device,
   - a distance adjustment step during which the distance between a right reference point of the right image to be seen by the right eye and a left reference point of the left image to be seen by the left eye is adjusted so that right reference point and left reference point provide parallel beams to the user,
   - a display parameter modifying step, during which at least one parameter of the display device is modified so as to modify the virtual display distance of the perceived image,
   wherein the display parameter modifying step is repeated until image subjective quality of the perceived image is perceived by the user as optimal, and
   - an eye parameter determining step during which an eye parameter is determined based on the parameter of the display device,
   wherein the image display step and the display parameter modifying step are implemented in binocular vision,
   wherein the binocular display device is configured to display independently images towards the two eyes of the user,
   wherein during the distance adjustment step the distance between the right reference point and the left reference point is adjusted based on the virtual convergence distance and
   the method further comprises:
   a scaling step during which the right and left images are scaled so that both images are seen with the same angular size by the user, and **characterized in that** a depth perception test step during which different images at different distances from a reference position are provided, and for each image it is checked if the user can detect if the distance is shorter or greater that the reference position.

2. The method according to claim 1, wherein the display device comprises moveable lenses through which the user sees the images displayed and during the display parameter modifying step the lenses are moved so as to modify the virtual display distance of the perceived image.

3. The method according to claim 1, wherein the display device comprises a light field display and during the display parameter modifying step at least one parameter of the light field display is modified so as to modify the virtual display distance of the perceived image.

4. The method according to claim 1, wherein the display device has means for selecting a virtual display distance among a set of predetermined virtual distances, and the virtual display distance of the perceived image is obtained from the use of at least one of the predetermined distance.

5. The method according to claim 1, wherein the display device has an optical element having adjusting means to adjust the power of the optical element.

6. The method according to any of the preceding claims, wherein the image subjective quality relates to the sharpness of the perceived image.

7. The method according to any of the preceding claims, wherein the method further comprises an astigmatism determining step during which an image or a plurality of images comprising similar elements with different orientations are displayed to the user and the orientations corresponding to the subjective sharpest perceived images are determined.

8. The method according to any of the preceding claims, wherein the perceived image subjective quality relates to the contrast of the perceived image.

9. The method according to any of the preceding claims, wherein the method further comprises an accommodation relaxation step during which the user's accommodation is relaxed.

**10.** The method according to any of the preceding claims, wherein the method further comprises a calibration step during which a correlation between the at least one parameter of the binocular display device modified during the display parameter modifying step and the virtual display distance is determined, said correlation is used during the eye parameter determining step to determine the eye parameter of the user.

**Patentansprüche**

**1.** Verfahren zum Bestimmen eines Augenparameters eines Benutzers einer Anzeigevorrichtung, wobei der Augenparameter einen dioptrischen Parameter einer ophthalmischen Linse betrifft, die für den Benutzer bereitzustellen ist, wobei das Verfahren Folgendes umfasst:

- einen Anzeigevorrichtungsbereitstellungsschritt, während dessen eine binokulare Anzeigevorrichtung für den Benutzer bereitgestellt wird,
- einen Bildanzeigeschritt, während dessen dem Benutzer ein Bild angezeigt wird, wenn dieser die Anzeigevorrichtung verwendet,
- einen Entfernungsanpassungsschritt, während dessen die Entfernung zwischen einem rechten Referenzpunkt des rechten Bildes, das durch das rechte Auge zu betrachten ist, und einem linken Referenzpunkt des linken Bildes, das durch das linke Auge zu betrachten ist, so angepasst wird, dass der rechte Referenzpunkt und linke Referenzpunkt parallele Strahlen zu dem Benutzer bereitstellen,
- einen Anzeigeparametermodifikationsschritt, während dessen wenigstens ein Parameter der Anzeigevorrichtung so modifiziert wird, dass die virtuelle Anzeigeentfernung zu dem wahrgenommenen Bild modifiziert wird, wobei der Anzeigeparametermodifikationsschritt wiederholt wird, bis eine subjektive Bildqualität des wahrgenommenen Bildes durch den Benutzer als optimal wahrgenommen wird, und
- einen Augenparameterbestimmungsschritt, während dessen ein Augenparameter basierend auf dem Parameter der Anzeigevorrichtung bestimmt wird,
wobei der Bildanzeigeschritt und der Anzeigeparametermodifikationsschritt in binokularer Sicht implementiert werden,
wobei die binokulare Anzeigevorrichtung zum unabhängigen Anzeigen von Bildern zu den zwei Augen des Benutzers hin konfiguriert ist,
wobei während des Entfernungsanpassungsschrittes die Entfernung zwischen dem rechten Referenzpunkt und dem linken Referenzpunkt basierend auf der virtuellen Konvergenzentfernung angepasst wird, und
das Verfahren ferner Folgendes umfasst:
einen Skalierungsschritt, während dessen das rechte und linke Bild so skaliert werden, dass beide Bilder durch den Benutzer mit der gleichen Winkelgröße gesehen werden, und **dadurch gekennzeichnet, dass** einen Tiefenwahrnehmungstestschritt, während dessen unterschiedliche Bilder in unterschiedlichen Entfernungen von einer Referenzposition bereitgestellt werden und für jedes Bild geprüft wird, ob der Benutzer detektieren kann, ob die Entfernung kleiner oder größer als die Referenzposition ist.

**2.** Verfahren nach Anspruch 1, wobei die Anzeigevorrichtung bewegliche Linsen umfasst, durch die der Benutzer die angezeigten Bilder betrachtet, und die Linsen während des Anzeigeparametermodifikationsschrittes so bewegt werden, dass die virtuelle Anzeigeentfernung des wahrgenommenen Bildes modifiziert wird.

**3.** Verfahren nach Anspruch 1, wobei die Anzeigevorrichtung eine Lichtfeldanzeige umfasst und wenigstens ein Parameter der Lichtfeldanzeige während des Anzeigeparametermodifikationsschrittes so modifiziert wird, dass die virtuelle Entfernung des wahrgenommenen Bildes modifiziert wird.

**4.** Verfahren nach Anspruch 1, wobei die Anzeigevorrichtung ein Mittel zum Auswählen einer virtuellen Anzeigeentfernung aus einem Satz vorbestimmter virtueller Entfernungen aufweist und die virtuelle Anzeigeentfernung des wahrgenommenen Bildes aus der Verwendung wenigstens einer der vorbestimmten Entfernungen erhalten wird.

**5.** Verfahren nach Anspruch 1, wobei die Anzeigevorrichtung ein optisches Element mit einem Anpassungsmittel zum Anpassen der Stärke des optischen Elements aufweist.

**6.** Verfahren nach einem der vorhergehenden Ansprüche, wobei die subjektive Bildqualität mit der Schärfe des wahrgenommenen Bildes zusammenhängt.

**7.** Verfahren nach einem der vorhergehenden Ansprüche, wobei das Verfahren ferner einen Astigmatismusbestimmungsschritt umfasst, während dessen dem Benutzer ein Bild oder mehrere Bilder, die ähnliche Elemente mit unterschiedlichen Orientierungen umfassen, angezeigt werden und die Orientierungen, die den subjektiv am schärfsten wahrgenommenen Bildern entsprechen, bestimmt werden.

8. Verfahren nach einem der vorhergehenden Ansprüche, wobei die subjektive wahrgenommene Bildqualität mit dem Kontrast des wahrgenommenen Bildes zusammenhängt.

9. verfahren nach einem der vorhergehenden Ansprüche, wobei das Verfahren ferner einen Akkommodationsentspannungsschritt umfasst, während dessen die Akkommodation des Benutzers entspannt wird.

10. Verfahren nach einem der vorhergehenden Ansprüche, wobei das Verfahren ferner einen Kalibrierungsschritt umfasst, während dessen eine Korrelation zwischen dem wenigstens einen Parameter der binokularen Anzeigevorrichtung, der während des Anzeigeparametermodifikationsschritts modifiziert wird, und der virtuellen Anzeigeentfernung bestimmt wird, wobei die Korrelation während des Augenparameterbestimmungsschrittes zum Bestimmen des Augenparameters des Benutzers verwendet wird.

**Revendications**

1. Procédé destiné à déterminer un paramètre oculaire d'un utilisateur d'un dispositif d'affichage, le paramètre oculaire concernant un paramètre dioptrique d'une lentille ophtalmique devant être fournie à l'utilisateur, le procédé comprenant :

   - une étape de fourniture de dispositif d'affichage, pendant laquelle un dispositif d'affichage binoculaire est fourni à l'utilisateur,
   - une étape d'affichage d'image, pendant laquelle une image est affichée pour l'utilisateur lorsqu'il utilise le dispositif d'affichage,
   - une étape de réglage de distance pendant laquelle la distance entre un point de référence droit de l'image droite devant être vue par l'œil droit et un point de référence gauche de l'image gauche devant être vue par l'œil gauche est réglée de telle sorte que le point de référence droit et le point de référence gauche fournissent des faisceaux parallèles à l'utilisateur,
   - une étape de modification de paramètres d'affichage, pendant laquelle au moins un paramètre du dispositif d'affichage est modifié de manière à modifier la distance d'affichage virtuelle de l'image perçue,
   dans lequel l'étape de modification de paramètres d'affichage est répétée jusqu'à ce qu'une qualité subjective d'image de l'image perçue soit perçue par l'utilisateur comme optimale, et
   - une étape de détermination de paramètre oculaire pendant laquelle un paramètre oculaire est déterminé sur la base du paramètre du dispositif d'affichage,
   dans lequel l'étape d'affichage d'image et l'étape de modification de paramètres d'affichage sont mises en œuvre en vision binoculaire,
   dans lequel le dispositif d'affichage binoculaire est conçu pour afficher indépendamment des images vers les deux yeux de l'utilisateur,
   dans lequel, pendant l'étape de réglage de distance, la distance entre le point de référence droit et le point de référence gauche est réglée sur la base de la distance de convergence virtuelle et
   le procédé comprend en outre :
   une étape de mise à l'échelle pendant laquelle les images droite et gauche sont mises à l'échelle de telle sorte que les deux images sont vues avec la même taille angulaire par l'utilisateur, et **caractérisé en ce qu'**une étape de test de perception de la profondeur pendant laquelle différentes images à différentes distances d'une position de référence sont fournies, et pour chaque image, il est vérifié si l'utilisateur peut détecter si la distance est plus courte ou plus longue que la position de référence.

2. Procédé selon la revendication 1, dans lequel le dispositif d'affichage comprend des lentilles mobiles à travers lesquelles l'utilisateur voit les images affichées, et pendant l'étape de modification de paramètres d'affichage, les lentilles sont déplacées de manière à modifier la distance d'affichage virtuelle de l'image perçue.

3. Procédé selon la revendication 1, dans lequel le dispositif d'affichage comprend un écran à champ lumineux, et pendant l'étape de modification de paramètres d'affichage, au moins un paramètre de l'écran à champ lumineux est modifié de manière à modifier la distance d'affichage virtuelle de l'image perçue.

**4.** Procédé selon la revendication 1, dans lequel le dispositif d'affichage comporte des moyens pour sélectionner une distance d'affichage virtuelle parmi un ensemble de distances virtuelles prédéterminées, et la distance d'affichage virtuelle de l'image perçue est obtenue à partir de l'utilisation d'au moins une des distances prédéterminées.

**5.** Procédé selon la revendication 1, dans lequel le dispositif d'affichage comporte un élément optique ayant des moyens de réglage pour régler la puissance de l'élément optique.

**6.** Procédé selon l'une quelconque des revendications précédentes, dans lequel la qualité subjective d'image concerne la netteté de l'image perçue.

**7.** Procédé selon l'une quelconque des revendications précédentes, dans lequel le procédé comprend en outre une étape de détermination d'astigmatisme pendant laquelle une image ou une pluralité d'images comprenant des éléments similaires avec des orientations différentes sont affichées pour l'utilisateur et les orientations correspondant aux images perçues subjectivement les plus nettes sont déterminées.

**8.** Procédé selon l'une quelconque des revendications précédentes, dans lequel la qualité subjective d'image perçue concerne le contraste de l'image perçue.

**9.** Procédé selon l'une quelconque des revendications précédentes, dans lequel le procédé comprend en outre une étape de relâchement de l'accommodation pendant laquelle l'accommodation de l'utilisateur est relâchée.

**10.** Procédé selon l'une quelconque des revendications précédentes, dans lequel le procédé comprend en outre une étape d'étalonnage pendant laquelle une corrélation entre l'au moins un paramètre du dispositif d'affichage binoculaire modifié pendant l'étape de modification de paramètres d'affichage et la distance d'affichage virtuelle est déterminée, ladite corrélation est utilisée pendant l'étape de détermination de paramètre oculaire pour déterminer le paramètre oculaire de l'utilisateur.

FIG. 1

FIG. 3

FIG. 4

FIG. 2

FIG. 5A

FIG. 5B

FIG. 6

FIG. 7A

FIG. 7B

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2016149416 A1

- US 20150016777 A